# FASCICULE DE BREVET EUROPEEN

(11) **EP 0 925 123 B1**
(45) Date de publication et mention de la délivrance du brevet: **19.12.2001**
(21) Numéro de dépôt: 97940218.7
(22) Date de dépôt: 11.09.1997
(51) Int. Cl.: B08B 1/00, A42B 3/26, B60S 1/04

(54) **ECRAN DE PROTECTION OPTIQUE A VISIBILITE AMELIOREE**
OPTISCHER SCHUTZSCHIRM MIT VERBESSERTER SICHT
OPTICAL PROTECTION SCREEN WITH IMPROVED VISIBILITY

(30) Priorité: 13.09.1996 FR 9611351; 28.05.1997 FR 9706721
(43) Date de publication de la demande: 30.06.1999
(73) Titulaire: Bouguerfa, Maxime, 54620 Baslieux (FR)
(72) Inventeur: Bouguerfa, Maxime, 54620 Baslieux (FR)
(74) Mandataire: Ballot, Paul Denis Jacques
(86) Numéro de dépôt international: FR9701604
(87) Numéro de publication internationale: WO9810877

(56) Documents cités:
- DE-A- 2 645 262
- DE-A- 3 512 109
- DE-A- 3 820 352
- FR-A- 1 258 079
- FR-A- 2 153 395
- US-A- 2 160 099

## Description

La présente invention concerne un écran de protection optique à visibilité améliorée, destiné à être placé dans le champ de vision d'un appareil optique ou d'une personne.

La projection de souillures, tels que la poussière ou la boue, provoque une diminution de la visibilité pouvant conduire à des situations dangereuses, notamment pour les conducteurs de motocyclettes.

On connaît un dispositif de protection constitué par un empilement de fines pellicules transparentes, placées généralement sur les visières de casques. Lorsque les pellicules sont sales, le conducteur les retire au fur et à mesure. On limite volontairement l'épaisseur de l'empilement pour éviter une distorsion du champ de vision. L'efficacité du dispositif est donc limitée dans le temps, du fait du nombre réduit de pellicules utilisables. De plus, le conducteur doit relâcher son attention en manipulant le dispositif pour retirer la pellicule salie, ce qui peut engendrer des accidents.

On connaît également un dispositif placé sur la visière d'un casque et composé d'un film transparent défilant devant la visière. Une fois que le film est entièrement utilisé, on le remplace en rechargeant le dispositif à la manière d'un appareil photographique. Lors du remplacement du film, ce dispositif nécessite des manipulations plus ou moins complexes et oblige l'utilisateur à transporter au moins un film de rechange, ce qui n'est pas très pratique à l'usage.

L'invention a pour objet un écran de protection optique permettant d'améliorer la visibilité du champ de vision en supprimant toutes les manipulations engendrées par un remplacement du film, en utilisant de nouveau un film nettoyé par l'intermédiaire d'un système de nettoyage intégré.

Avec ces objectifs en vue, l'invention a pour objet un écran de protection optique à visibilité améliorée comportant des moyens d'entraînement pour mettre en mouvement, dans une direction de déplacement, un film transparent placé dans un champ de vision d'un appareil optique ou d'une personne, et comportant des éléments de nettoyage, constitués par des balais, qui s'étendent transversalement à la direction de déplacement de la feuille, en s'appliquant contre la dite feuille, et sont situés sur au moins un coté du champ de vision, caractérisé en ce qu'il comporte un cadre dans lequel est pratiquée une fenêtre, le cadre comportant deux boitiers situés à proximité des cotés latéraux de la fenêtre, et le film se déplaçe en translation dans la fenêtre entre les deux boitiers, alternativement dans un sens puis dans un autre, sous l'action de moyens d'entraînement adaptés pour mettre en mouvement le film, les balais étant situés de chaque coté de la fenêtre.

Lors du déplacement de la feuille, les balais frottent contre le film pour assurer l'enlèvement des salissures et rendre le film propre et net. Ainsi, on supprime le remplacement systématique du film usagé en nettoyant le film pour l'utiliser de nouveau, ce qui augmente considérablement sa durabilité et destine particulièrement bien l'écran pour un usage dans des conditions d'utilisations difficiles.

Le nettoyage s'obtient par contact des balais avec le film, conjugué avec le déplacement alternatif du film. On améliore également le nettoyage en utilisant plusieurs balais disposés de chaque coté de la fenêtre.

Selon une première variante de réalisation, l'écran comporte deux bobines creuses, situées respectivement dans les deux boitiers, autour desquelles s'enroule et se déroule le film.

L'utilisation de bobines creuses permet d'y loger les moyens d'entraînement. De ce fait, on réduit l'encombrement général de l'écran ainsi que le poids en proposant un entraînement ne nécessitant pas de nombreuses pièces.

Selon une deuxième variante de réalisation, le cadre comporte des parties de stockage situées sur les boitiers.

Dans cette variante, on utilise une partie de stockage pour loger le film qui se déplace alors en coulissant à l'intérieur de la dite partie, au lieu de s'enrouler autour d'une bobine. Par exemple, cette partie de stockage peut être constituée par des branches de maintien de l'écran sur un casque de motocycliste.

Selon un mode de réalisation préféré, les moyens d'entraînement sont constitués par un moteur électrique placé coaxialement dans une première des dites bobines, la dite première bobine étant reliée par l'intermédiaire du film à une deuxième bobine pourvue d'un ressort de rappel situé sur un support qui est placé coaxialement dans la dite deuxième bobine.

L'utilisation d'une motorisation électrique pour déplacer le film permet d'éviter toute manipulation manuelle qui provoque inévitablement un relâchement de l'attention de l'utilisateur. En fonctionnement, le moteur électrique entraîne la première bobine autour de laquelle s'enroule une extrémité du film, l'autre extrémité du film se déroulant alors de la deuxième bobine. Un ressort, préférentiellement un ressort de torsion, est placé coaxialement à l'intérieur de la deuxième bobine et se comprime lorsque le film se déroule de la dite bobine.

Ensuite, lorsqu'on déroule le film de la première bobine en inversant le sens de rotation du moteur, le ressort se détend pour assurer l'enroulement automatique du film autour de la deuxième bobine. Comme on peut le constater, ce moyen d'entraînement communique facilement un mouvement alternatif au film. Le ressort assure également, pendant le déplacement du film, une légère tension du dit film.

Selon une autre variante de réalisation, les boitiers contiennent un liquide de nettoyage et des moyens d'étanchéité, pour éviter toute fuite du dit liquide, sont constitués par les balais qui sont situés de part et d'autre du film.

L'utilisation conjuguée du liquide de nettoyage et des balais permet d'améliorer notablement la netteté et la propreté du film. On peut utiliser par exemple un liquide permettant la dissolution des souillures déposées sur le film. Une fois que le produit a fait son effet, les balais essuient alors l'excédent de liquide présent sur le film. Pour éviter toute fuite de liquide de nettoyage, les balais assurent l'étanchéité des boitiers.

Selon une disposition particulière, les balais sont inclinés par rapport à la direction de défilement du film.

Dans cette disposition, l'inclinaison des balais permet de donner une direction préférentielle d'évacuation des souillures. Lors du déplacement du film, les souillures présentes sur le film viennent chasser les souillures déjà accumulées sur les balais et du fait de l'inclinaison des balais, les souillures accumulées sont alors évacuées plus efficacement.

Selon un mode de réalisation préféré, les balais s'escamotent lorsque le film se déplace dans un sens puis reviennent à son contact lorsque le film se déplace dans l'autre sens pour essuyer l'excédent de liquide de nettoyage et l'écran comporte des moyens d'étanchéité constitués par des lèvres placées transversalement à la direction de déplacement du film et venant pincer légèrement le film.

Cette disposition permet d'éviter que, lors de l'enroulement du film, les souillures présentes sur le film s'accumulent au niveau des balais du coté de la fenêtre, comme cela pourrait être le cas si les balais sont maintenu en permanence contre le film. Dans un tel cas, lors du déroulement, le film risquerait d'entraîner cette accumulation, et il en résulterait l'apparition de traces néfastes pour la visibilité. Conformément au mode de réalisation, pour éviter l'apparition de telles traces, on escamote les balais situés du coté où s'enroule le film afin de ne pas racler les souillures lors de l'enroulement mais, on ramène ensuite les balais au contact du film, lors du déroulement du film, pour retenir les souillures du coté des balais situé vers la bobine et essuyer l'excédent de liquide de nettoyage. De ce fait, les souillures ne risquent pas de revenir dans le champ de vision, et n'ont pas non plus tendance à s'agglomérer sur les balais puisque le liquide assure le nettoyage du film avant que les balais n'exercent leur fonction de raclage.

D'autres avantages et caractéristiques de l'invention apparaîtront dans la description qui va suivre d'un écran de protection à visibilité améliorée conforme à l'invention, destiné en particulier à être placé sur un casque de motocycliste.

On se rapportera aux différents dessins annexés dans lesquels :
- la figure 1 est une vue d'ensemble en perspective montrant un écran de protection rapporté sur un casque de motocycliste,
- la figure 2 est une vue de face éclatée de l'écran montrant notamment les moyens d'entraînement et les moyens de stockage du film sous forme de bobines,
- la figure 3 est une vue de dessus du cadre de l'écran, un couvercle ayant été préalablement enlevé, illustrant en particulier le montage des balais qui assurent le nettoyage du film,
- la figure 4 est une vue en perspective montrant une variante de réalisation des moyens de stockage du film,
- la figure 5 est une vue du mécanisme d'entraînement du film correspondante à la figure 2, dans le cas d'une variante utilisant deux moteurs électriques pour entraîner le film,
- la figure 6 est une vue schématique en perspective illustrant une variante dans laquelle les balais sont inclinés pour améliorer l'évacuation des souillures,
- la figure 7 est une vue partielle en coupe selon la ligne VII-VII de la figure 2, montrant une variante préférentielle de réalisation utilisant des balais escamotables,
- la figure 8 est une vue en schématique perspective montrant une variante illustrant la réalisation de canaux d'évacuation,
- la figure 9 est une vue schématique de face illustrant une variante de réalisation présentant un écran de protection sous la forme d'une paire de lunette et utilisant des feuilles en forme de disque.

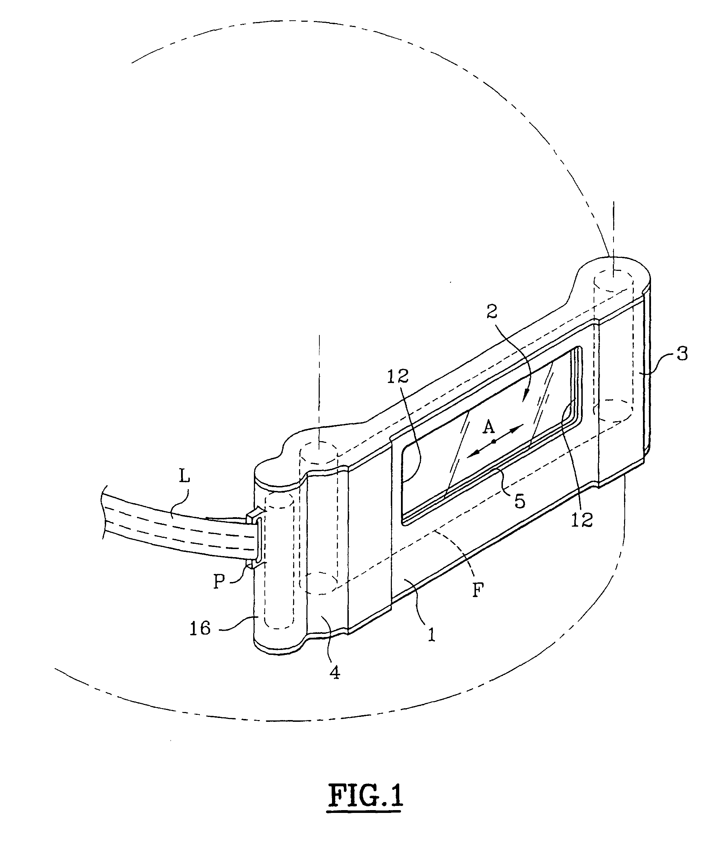

L'écran de protection représenté figure 1 se compose d'un cadre 1 dans lequel est pratiquée une fenêtre 2, par exemple de forme rectangulaire, qui est située dans le champ de vision d'une personne, par exemple sur la visière d'un casque d'un conducteur de motocyclette. L'écran est maintenu en position sur le casque par une lanière élastique L qui est solidarisée sur le cadre 1 par l'intermédiaire de pattes de fixation P.

Des orifices sont réalisés dans le cadre 1 pour former un premier boitier 3 et un second boitier 4 qui sont situés de part et d'autre des cotés latéraux 12 de la fenêtre 2. Un film F transparent se déplace alternativement vers un boitier puis vers l'autre, selon une direction de déplacement (flèche A) et une rainure 5 est pratiquée dans le cadre 1 pour assurer le guidage du film dans la fenêtre 2.

L'écran est montré sur la figure 1 dans une position d'utilisation où la direction de déplacement du film est horizontale. L'écran peut aussi bien fonctionner dans une position verticale, c'est à dire que l'orientation du boitier serait perpendiculaire à la direction horizontale de déplacement, si nécessaire.

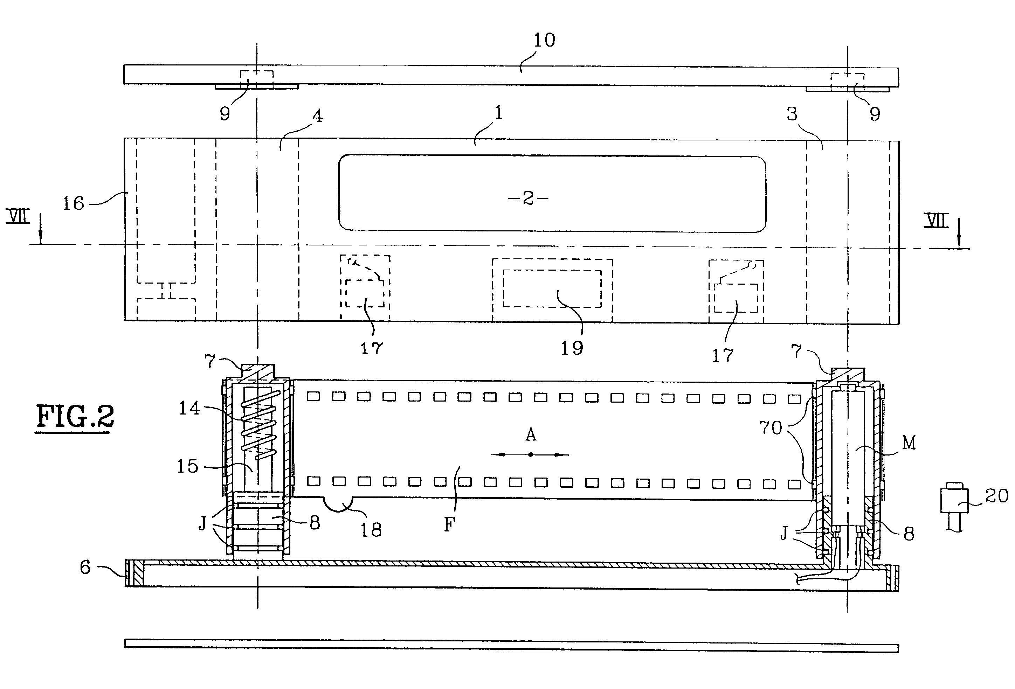

Comme le montre la figure 2, le cadre comporte une semelle 6 qui porte des bobines creuses 7. En fonctionnement, le film F s'enroule et se déroule autour de ces bobines qui s'étendent perpendiculairement à la direction de déplacement du film et qui viennent se loger à l'intérieur des boitiers 3, 4.

Dans ce cas, on choisira le film dans un matériau présentant une certaine souplesse, par exemple le polycarbonate, pour assurer un enroulement correct du film autour des bobines.

Le guidage en rotation des bobines est assuré par des tourillons 8 solidaires de la semelle 6 et qui s'insèrent dans l'une des extrémités des bobines 7 tandis que l'autre extrémité vient se loger dans des évidements 9 réalisés dans un couvercle 10.

Le film F est déplacé sous l'action de moyens d'entraînement constitués par un moteur électrique M qui est placé coaxialement dans une première des bobines 7 et la dite première bobine est reliée par l'intermédiaire du film à une deuxième bobine pourvue d'un ressort de rappel 14 situé sur un support 15 qui est placé coaxialement dans la dite deuxième bobine. Une des extrémités du ressort 14 est fixée sur la deuxième bobine et l'autre extrémité est fixée sur le support 15.

L'entraînement du film s'obtient, de manière connue de l'homme du métier, par des dents 70 solidaires des bobines 7 et qui engrènent respectivement avec deux séries d'encoches parallèles réalisées au niveau des bords latéraux du film, à la manière d'un film photographique.

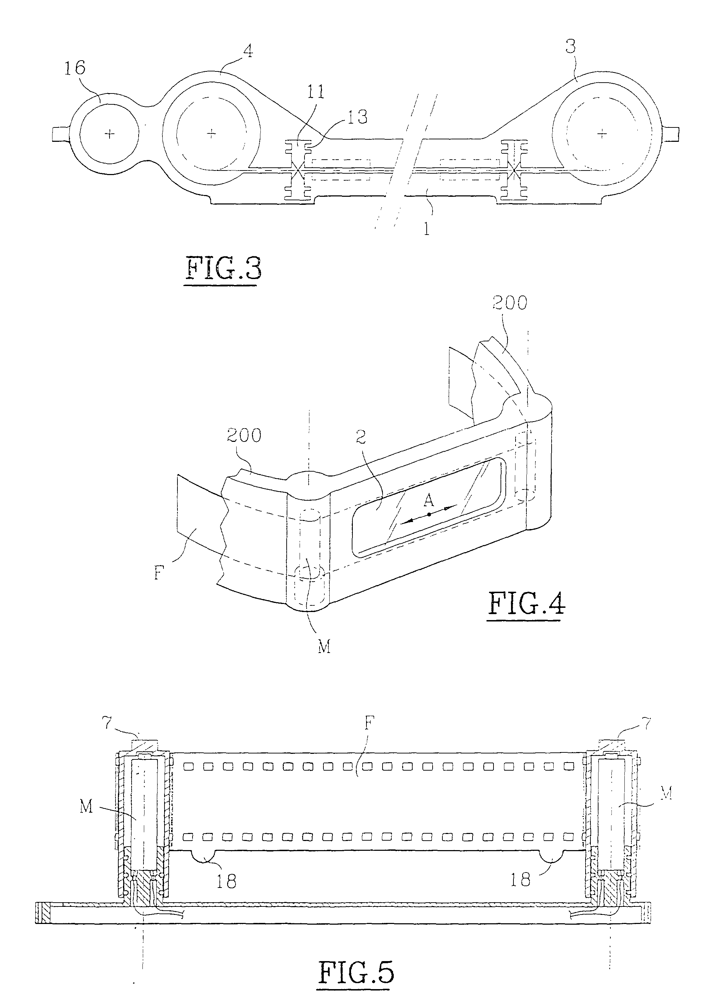

Le nettoyage du film s'obtient par la présence d'éléments de nettoyage, montrés figure 3, constitués par des balais 11, de type balais d'essuie-glace, qui viennent en contact avec le film F.

Ces balais 11 sont disposés transversalement à la direction de déplacement du film, de part et d'autre du dit film et à proximité directe des cotés latéraux 12 de la fenêtre 2. Les balais 11 sont placés dans des rainures 13 et sont amovibles pour permettre un remplacement facile des balais usés mais également pour utiliser d'autres types de balais, tels que des brosses, des feutres ou des éponges pour adapter les moyens de nettoyage aux conditions d'utilisation de l'écran. Il est également possible d'utiliser un liquide de nettoyage contenu dans les boitiers 3, 4 pour améliorer l'efficacité du nettoyage, en complément de l'action des balais 11.

Dans ce cas, pour éviter une fuite de liquide de nettoyage, l'étanchéité des boitiers 3, 4 est assurée par la présence des balais 11 qui sont au contact du film figure 3) et des joints toriques J (figure 2) seront placés entre les tourillons 8 et les bobines 7 pour protéger le moteur électrique M de la pénétration du liquide de nettoyage.

L'association des rainures 5 qui guident le film F et des balais 11 qui sont en contact avec le film F assure une immobilisation et une légère tension du dit film lorsque les moyens d'entraînement du film sont à l'arrêt, permettant ainsi d'éviter un déroulement entier des bobines, par exemple lorsque le vent s'engouffre dans la fenêtre 2 quand l'utilisateur se déplace à grande vitesse avec l'écran. De plus, à l'arrêt, le moteur électrique M assure le blocage en rotation de la première des bobines 7, ce qui permet d'immobiliser une extrémité du film. L'autre extrémité du film est soumise à un effort induit du couple exercé par le ressort 14 sur la deuxième bobine, le dit effort permettant de tendre le film F.

Le moteur électrique est alimenté par une batterie (non représentée) qui est logée dans un compartiment 16 constitué par un orifice réalisé dans le cadre 1.

Un interrupteur 20, accessible de l'extérieur du cadre 1, est prévu pour arrêter le déplacement du film en coupant l'alimentation électrique de la batterie.

On va maintenant décrire en détail le principe de fonctionnement de l'écran de protection et en particulier le nettoyage du film F conforme à l'invention.

Comme on l'a déjà précisé, lors de mauvaises conditions climatiques, des projections diverses peuvent venir souiller le film F et ainsi réduire la visibilité. L'utilisateur peut commencer le nettoyage du film en actionnant l'interrupteur 20, ce qui permet à la batterie d'alimenter le moteur électrique M.

Comme le montre la figure 2, le moteur électrique M qui provoque le déplacement du film, est commandé par un circuit de commande composé d'interrupteurs à levier 17 conformés pour coopérer avec un ergot 18 réalisé sur le film F et d'un relais 19. Ce circuit de commande est situé à l'intérieur du cadre 1 et permet d'assurer le déplacement alternatif sans interruption du film F. L'ergot 18 est en contact avec un premier des interrupteurs 17.

Le moteur électrique M entraîne alors la première des bobines 7 autour de laquelle s'enroule le film F. La deuxième bobine est alors entraînée en rotation par l'intermédiaire de la liaison du film à la première bobine, permettant un déroulement du film de la dite bobine et une compression du ressort 14.

Lors du déplacement, l'ergot 18 actionne un deuxième des interrupteurs à levier 17 en agissant comme une came, le dit interrupteur commandant le relais 19 pour inverser l'alimentation électrique du moteur, ce qui modifie alors le sens de rotation du dit moteur. A ce moment, le film se déroule de la première bobine qui est entraînée par le moteur et le ressort 14, en se détendant, entraîne en rotation la deuxième bobine pour permettre l'enroulement du film autour de la dite deuxième bobine. Ainsi, lors de ce déplacement, l'ergot 18 rencontre de nouveau le premier interrupteur qui commande le relais 19 pour inverser l'alimentation du moteur M et recommencer ainsi le cycle de déplacement du film.

L'utilisateur peut à tout moment interrompre le déplacement du film en actionnant simplement l'interrupteur 20.

De plus, le circuit de commande peut être réalisé pour assurer une variation de vitesse de défilement du film, lors d'un déplacement continu ou intermittent, suivant les conditions d'utilisation de l'écran.

La figure 4 présente une variante de réalisation où le film F, en se déplaçant, coulisse à l'intérieur d'une partie de stockage 200, telle que les branches qui servent au maintien de l'écran sur le casque de l'utilisateur. Le déplacement alternatif du film selon la direction de déplacement (flèche A) est obtenu en inversant le sens de rotation d'au moins un moteur M qui engrène avec le film F. Le nettoyage du film s'obtient par l'action combinée des balais 11 avec le déplacement du film, comme décrit précédemment.

La figure 5 présente une autre variante dans laquelle on utilise deux moteurs électriques M placés à l'intérieur des premières et deuxièmes bobines 7. Le circuit de commande sera réalisé de manière à commander simultanément les deux moteurs pour assurer l'enroulement du film F autour de la première bobine et le déroulement de la deuxième bobine et inversement, dès que l'interrupteur à levier 17 est actionné, dérouler le film de la première bobine et l'enrouler autour de la deuxième bobine.

Il est également possible, lors de l'enroulement ou du déroulement, de commander les deux moteurs pour tourner à des vitesses légèrement différentes, la bobine entraînée à vitesse réduite créant alors un effort résistant pour la bobine entraînante, ce qui assure la tension du film pendant le déplacement.

Pour réaliser la tension du film F à l'arrêt, le circuit de commande sera réalisé pour commander les moteurs M de manière à ce que chaque bobine 7 enroule le film, en créant des couples d'enroulement avec des valeurs identiques mais des sens opposés. Ainsi, des efforts opposés qui sont issus des dits couples, seront appliqués à chaque extrémité du film pour assurer la tension.

De plus, on peut également prévoir une variation de la vitesse des deux bobines de manière à assurer une vitesse constante de défilement du film pour pallier aux variations de rayons des bobines, consécutives à l'enroulement ou au déroulement du film, qui engendrent une variation des vitesses tangentielles des bobines 7.

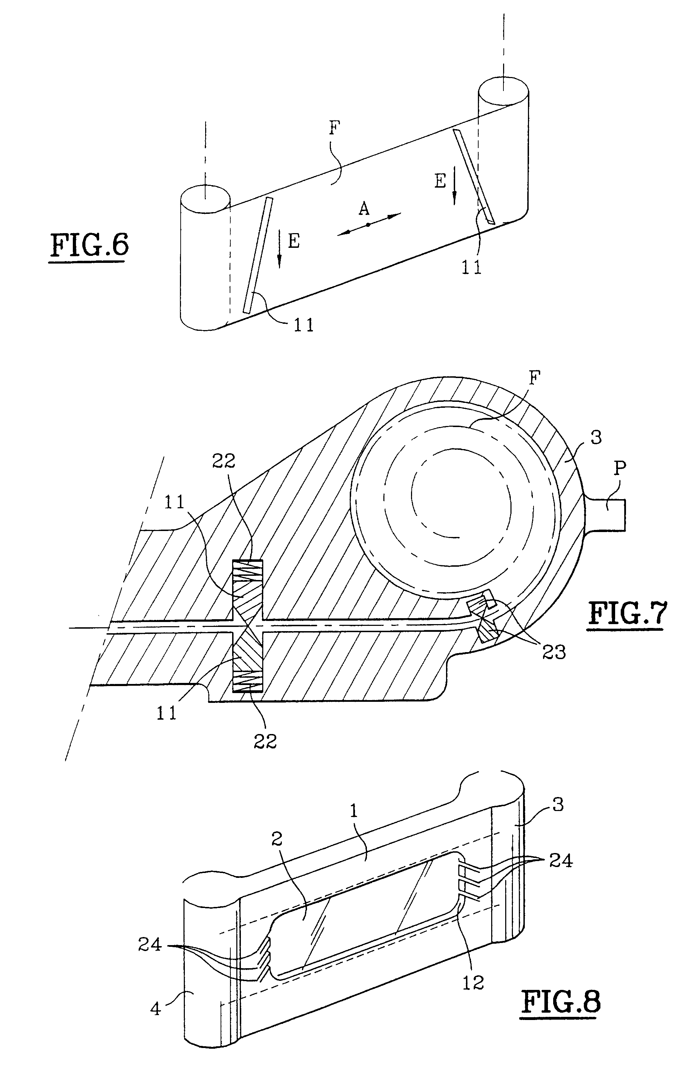

Comme le montre la figure 6, on peut évacuer plus efficacement, lors du nettoyage, les souillures présentes au niveau des balais ou l'excès de liquide nettoyage, en donnant une direction préférentielle d'évacuation (flèche E) en inclinant les balais 11 par rapport à la direction de déplacement du film. Dans ce cas, au fur et à mesure du déplacement du film, les souillures présentes sur le film viennent chasser les souillures déjà accumulées sur les balais 11. Ces souillures accumulées ont une tendance naturelle, du fait de la combinaison de l'inclinaison des balais avec la direction de déplacement, à s'évacuer vers le bas. Cette disposition permet d'éviter une accumulation trop importante des souillures au niveau des balais 11, ce qui nuirait à la propreté du film.

Egalement dans ce but, on peut escamoter les balais 11, comme le présente la figure 7. Une came (non représentée), solidaire du film, a un profil conformé pour coopérer avec les balais 11. Lorsque le film se déplace, la came provoque l'escamotage des balais situés du coté où s'enroule le film F en dégageant les balais pour éviter tout contact avec le film puis on ramène ensuite les balais au contact du film, lors du déroulement, par l'intermédiaire des ressorts 22. Le nettoyage du film est obtenu par du liquide de nettoyage contenu dans les boitiers 3, 4 et les balais 11 n'assurent alors que l'essuyage de l'excédent de liquide de nettoyage présent sur le film F.

Pour éviter une fuite de liquide de nettoyage lorsque les balais 11 s'escamotent, on assure l'étanchéité des boitiers 3, 4 en utilisant des lèvres 23 qui sont placées transversalement par rapport à la direction de déplacement du film et qui viennent pincer légèrement le film F, comme le montre la figure 7.

Toujours pour éviter une accumulation importante de souillures au niveau des balais, comme le montre la figure 8, on prévoit également des canaux 24 situés sur les bords 12 de la fenêtre 2. Ces canaux permettent d'évacuer vers l'extérieur de l'écran une accumulation en excès de la souillure qui ne pourrait pas être nettoyée par les balais 11.

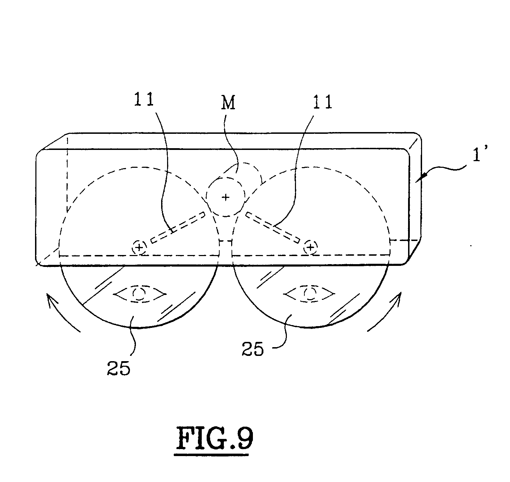

Dans la variante de réalisation montrée figure 9, on utilise une feuille en forme de disque 25. L'écran se présente alors sous la forme d'une paire de lunette comportant un cadre 1', telle qu'une boite de forme parallélépipèdique, comportant deux disques 25. Les deux disques 25 d'axes parallèles ont une moitié située à l'extérieur du cadre 1', pour être par exemple placée devant les yeux d'un utilisateur, et une moitié située à l'intérieur du cadre 1'. Un moteur électrique M, s'étendant selon les axes des disques, est placé dans un espace situé entre les deux dits disques 25. Le moteur entraîne en rotation continue de sens contraire les dits disques, par l'intermédiaire d'un pignon (non représenté) conformé pour engrener avec des dents (non représentées) situées sur un bord périphérique des disques 25. Les balais 11 sont alors disposés selon un rayon du disque pour assurer le nettoyage et le cadre 1 peut contenir un liquide de nettoyage en complément de l'action des balais.

Les disques seront choisis dans un matériau assez rigide, tel que le PVC, pour éviter toute déformation. Il est possible également d'entraîner les disques 25 pour assurer un déplacement angulaire alternatif, la partie du disque nettoyée revenant alors dans le champ de vision. De plus dans ce cas, on peut utiliser une portion de disque à la place du disque complet, ceci permettant de reduire l'encombrement de l'écran et surtout le poids.

L'invention ne se limite pas aux différents modes de réalisation décrits ci-dessus uniquement à titre d'exemple.

En particulier :
- L'écran de protection peut être disposé sur un élément de protection existant, tel que sur un pare-brise, le dit écran assurant le nettoyage des souillures,
- L'écran de protection peut être amovible et on peut alors utiliser une fixation adéquate, telle que des ventouses, pour permettre, par exemple, un relèvement possible de la visière d'un casque.
- L'écran pourra être conformée pour épouser la forme du champ de vision, tel que par exemple un cintrage de l'écran pour l'appliquer sur la visière bombée d'un casque.
- La forme en disque ou portion de disque de la feuille (dans le cas de la variante de la figure 9) n'est pas limitée, en particulier la feuille peut être de forme elliptique ou autre.
- L'interrupteur à levier 17 du circuit de commande pourra être aisément remplacé par une cellule photo-électrique, un photo-transistor ou tout autre dispositif mécanique, optique ou électronique pouvant réaliser la détection du passage du profil 18.
- Les balais 11 pourront ne pas être disposés à proximité des cotés latéraux 12 de la fenêtre 2 mais déplacés du coté des boitiers 3, 4.
- Le film F pourra posséder plusieurs zones dont les dimensions seront égales aux dimensions de la fenêtre 2 et sur lesquelles on pourra appliquer différents traitements mécaniques ou chimiques spécifiques pour améliorer les qualités du film, tels que les traitements anti-rayures, anti-buée, de polarisation de la lumière, de teinte du film, c'est à dire tout ce qui permet d'adapter l'écran de protection en fonction des conditions climatiques. Le circuit de commande devra alors se composer d'un circuit intégré programmable conformé pour gérer le déplacement du film en plaçant les différentes zones dans la fenêtre 2 selon le souhait de l'utilisateur.
- Les dents 70 qui engrènent avec les encoches du film F pourront être aisément remplacées par des fentes réalisées dans les bobines selon une direction axiale dans lesquelles viennent pénétrer les extrémités du film ou par un morceau de ruban adhésif qui déborderait des extrémités du film pour être collé sur la bobine.
- il est possible de remplacer l'entraînement électrique du film F par un entraînement mécanique, tel qu'un mécanisme à cliquet commandé en tirant latéralement une ficelle,
- L'écran de protection (dans le cas de la variante présentée figure 9) pourra ne comporter qu'un seul disque.
- L'écran de protection, avec une feuille sous la forme d'un film ou d'un disque, pourra s'utiliser dans des applications susceptibles d'une diminution de visibilité néfaste pour la sécurité, telles que sur les visières de casques ou les lunettes, les caméras, les appareils photographiques, les masques, les optiques de phares, pare-brise et rétroviseurs de véhicule automobile ou intégré dans les capots transparents qui protègent les machines-outils utilisant un système d'arrosage aspergeant du fluide de coupe

## Revendications

1. Ecran de protection optique à visibilité améliorée, placé sur un appareil optique ou sur un élément de protection d'une personne, comportant
deux boîtiers (3, 4),
un film (F) transparent qui se déplace en translation entre les deux boîtiers (3, 4),
des éléments de nettoyage, constitués par des balais (11), qui s'étendent transversalement à la direction du film (F), qui sont situés à proximité d'au moins un des boîtiers (3, 4) et qui s'appliquent contre ledit film (F),
des moyens d'entraînement (M, 14) adaptés pour mettre en mouvement le film (F),
**caractérisé en ce que** l'écran comporte en outre un cadre (1) supportant les deux boîtiers et dans lequel est pratiquée une fenêtre (2) entre les deux boîtiers, le film se déplaçant dans la fenêtre, le cadre étant placé de manière amovible sur l'appareil optique ou sur l'élément de protection d'une personne pour que la fenêtre soit dans un champ de vision de l'appareil optique ou de la personne, et
le film (F) se déplace alternativement dans un sens puis dans l'autre.

2. Ecran selon la revendication 1, **caractérisé en ce qu'**il comporte deux bobines (7), situées respectivement dans les deux boitiers (3, 4), autour desquelles s'enroule et se déroule le film (F).

3. Ecran selon la revendication 1, **caractérisé en ce que** le cadre comporte des parties de stockage (200) situées sur les boitiers (3, 4).

4. Ecran selon la revendication 2, **caractérisé en ce que** les bobines (7) sont creuses et les moyens d'entraînement sont constitués par un moteur électrique (M) placé coaxialement dans une première des dites bobines (7), la dite première bobine étant reliée par l'intermédiaire du film (F) à une deuxième bobine pourvue d'un ressort de rappel (14) situé sur un support (15) qui est placé coaxialement dans la dite deuxième bobine.

5. Ecran selon la revendication 2, **caractérisé en ce que** les bobines (7) sont creuses et les moyens d'entraînement sont constitués par deux moteurs électriques (M) placés coaxialement dans chaque bobine (7).

6. Ecran selon la revendication 1, **caractérisé en ce que** les boitiers (3, 4) contiennent un liquide de nettoyage et des moyens d'étanchéité, pour éviter toute fuite du dit liquide, sont constitués par les balais qui sont situés de part et d'autre du film.

7. Ecran selon la revendication 2, **caractérisé en ce que** les balais (11) sont inclinés par rapport à la direction de défilement (A) du film.

8. Ecran selon la revendication 6, **caractérisé en ce que** les balais (11) s'escamotent lorsque le film (F) se déplace dans un sens puis reviennent à son contact lorsque le film se déplace dans l'autre sens pour essuyer l'excédent de liquide de nettoyage et **en ce que** les moyens d'étanchéité, constitués par des lèvres (23), sont placés transversalement à la direction de déplacement (A) du film (F) et viennent pincer légèrement le dit film.

9. Ecran selon la revendication 1, **caractérisé en ce que** les boitiers (3, 4) comportent un moyen d'évacuation de la salissure (24) situé sur les cotés latéraux (12) de la fenêtre (2).

## Patentansprüche

1. Optischer Schutzschirm mit verbesserter Sicht, der auf einem optischen Gerät oder einem Schutzelement für eine Person angebracht ist, und der aufweist
- zwei Gehäuse (3, 4),
- einen durchsichtigen Film (F), der sich in Translationsrichtung zwischen den beiden Gehäusen (3,4 ) verschiebt,
- Reinigungselemente, die aus Wischern (11) bestehen, welche sich quer zur Richtung des Films (F) erstrecken, in der Nähe mindestens eines der Gehäuse (3, 4) angeordnet sind und sich gegen den Film (F) anlegen,
- Antriebsmittel (M, 14), die geeignet sind, um den Film (F) in Bewegung zu versetzen,
**dadurch gekennzeichnet, daß** der Schirm außerdem einen Rahmen (1) aufweist, der die beiden Gehäuse trägt und in den ein Fenster (2) zwischen den beiden Gehäusen eingearbeitet ist, wobei der Film sich im Fenster verschiebt, wobei der Rahmen lösbar auf dem optischen Gerät oder dem Schutzelement für eine Person angeordnet ist, damit das Fenster sich in einem Sichtfeld des optischen Geräts oder der Person befindet, und
der Film (F) sich abwechselnd in der einen und dann in der anderen Richtung verschiebt.

2. Schirm nach Anspruch 1, **dadurch gekennzeichnet, daß** er zwei sich je in einem der beiden Gehäuse (3, 4) befindende Spulen (7) aufweist, um die sich der Film (F) auf- und abwickelt.

3. Schirm nach Anspruch 1, **dadurch gekennzeichnet, daß** der Schirm Lagerbereiche (200) aufweist, die sich auf den Gehäusen (3, 4) befinden.

4. Schirm nach Anspruch 2, **dadurch gekennzeichnet, daß** die Spulen (7) hohl sind, und die Antriebsmittel aus einem Elektromotor (M) bestehen, der koaxial in einer ersten Spule (7) angeordnet ist, wobei die erste Spule über den Film (F) mit einer zweiten Spule verbunden ist, die mit einer Rückholfeder (14) versehen ist, welche sich auf einem Träger (15) befindet, der koaxial in der zweiten Spule angeordnet ist.

5. Schirm nach Anspruch 2, **dadurch gekennzeichnet, daß** die Spulen (7) hohl sind, und die Antriebsmittel aus zwei Elektromotoren (M) bestehen, die koaxial in jeder Spule (7) angeordnet sind.

6. Schirm nach Anspruch 1, **dadurch gekennzeichnet, daß** die Gehäuse (3, 4) eine Reinigungsflüssigkeit enthalten, und daß Abdichtmittel, um jedes Auslaufen der Flüssigkeit zu vermeiden, aus den Wischern bestehen, die zu beiden Seiten des Films angeordnet sind.

7. Schirm nach Anspruch 2, **dadurch gekennzeichnet, daß** die Wischer (11) in bezug auf die Vorbeilaufrichtung (A) des Films geneigt sind.

8. Schirm nach Anspruch 6, **dadurch gekennzeichnet, daß** die Wischer (11) eingezogen werden, wenn der Film (F) sich in einer Richtung verschiebt, und dann wieder in Kontakt mit dem Film kommen, wenn er sich in die andere Richtung verschiebt, um den Überschuß an Reinigungsflüssigkeit abzuwischen, und daß die aus Lippen (23) bestehenden Abdichtmittel quer zur Verschieberichtung (A) des Films (F) angeordnet sind und den Film leicht einklemmen.

9. Schirm nach Anspruch 1, **dadurch gekennzeichnet, daß** die Gehäuse (3, 4) ein Mittel (24) zum Entfernen der Verschmutzung aufweisen, das sich auf den Seitenrändern (12) des Fensters (2) befindet.

## Claims

1. An optical protection screen with improved visibility, placed on an optical apparatus or on a personal protection element, and comprising:
two housings (3, 4),
a transparent film (F) which is displaced in straight line motion between the two housings (3, 4),
cleaning elements consisting of wipers (11), which extend transversely to the direction of the film (F), are situated close to at least one of the housings (3, 4), and are applied against the said film (F),
driving means (M, 14) adapted to put the film (F) in motion,
**characterised in that** the screen further includes a frame
(1) which supports the two housings and in which a window (2) is formed between the two housings, the film being displaced in the window, the frame being placed removably on the optical apparatus or personal protection element, so that the window is in the field of vision of the optical apparatus or of the person protected, and the film (F) is displaced alternately, in one direction and then in the other.

2. A screen according to Claim 1, **characterised in that** it includes two spools (7), situated respectively in the two housings (3, 4) and around which the film (F) is wound and unwound.

3. A screen according to Claim 1, **characterised in that** the frame includes storage portions (200) situated on the housings (3, 4).

4. A screen according to Claim 2, **characterised in that** the spools (7) are hollow, and the driving means comprise an electric motor (M) which is located coaxially in a first one of the said spools (7), the said first spool being connected by the film (F) to a second spool which is provided with a return spring (14), situated on a support (15) which is located coaxially in the said second spool.

5. A screen according to Claim 2, **characterised in that** the spools (7) are hollow, and the driving means consist of two electric motors (M), located coaxially in each spool (7).

6. A screen according to Claim 1, **characterised in that** the housings (3, 4) contain a cleaning liquid, and sealing means, for preventing all leakage of the said liquid, consist of the wipers which are located on either side of the film.

7. A screen according to Claim 2, **characterised in that** the wipers (11) are inclined with respect to the direction of movement (A) of the film.

8. A screen according to Claim 6, **characterised in that** the wipers (11) retract when the film is displaced in one direction, and then return to their contact when the film is displaced in the other direction, whereby to sweep off the excess of cleaning liquid, and **in that** the sealing means, consisting of lips (23), are placed transversely to the direction of movement (A) of the film (F), and slightly pinch the said film.

9. A screen according to Claim 1, **characterised in that** the housings (3, 4) include a dirt evacuation means (24), situated on the lateral sides (12) of the window (2).
